# DEMANDE DE BREVET EUROPEEN

(11) **EP 2 923 680 A1**
(43) Date de publication de la demande: **30.09.2015**
(21) Numéro de dépôt: 15160467.5
(22) Date de dépôt: 24.03.2015
(51) Int. Cl.: A61F 5/56

(54) **DISPOSITIF D'OCCLUSION DENTAIRE POUR LA SUPPRESSION DE L'INTENSITÉ DES FORCES DE BRUXISME**

(30) Priorité: 25.03.2014 FR 1452536
(71) Demandeur: Caparros, Charles, 65240 Avajan (FR)
(72) Inventeur: Caparros, Charles, 65240 Avajan (FR)
(74) Mandataire: Fragnaud, Aude

(57) **Abrégé**

L'invention concerne un dispositif d'occlusion dentaire pour la suppression de l'intensité des forces de bruxisme. Selon l'invention le dispositif 100 comprend un corps (20) comportant un matériau souple caoutchouteux, ledit corps se présentant sous la forme d'un pavé et ayant une forme adaptée pour occuper un espace entre la denture supérieure et la denture inférieure et rendre impossible tout contact direct entre les deux dentures.

## Description

### [Domaine de l'invention]

La présente invention concerne un dispositif d'occlusion dentaire pour la suppression de l'intensité des forces de bruxisme.

De nombreuses personnes souffrent de symptômes provoqués par un phénomène consistant à serrer et / ou grincer les dents pendant le sommeil et cela de façon inconsciente. Ces mouvements répétitifs inconscients, de la mâchoire sont appelés bruxisme.

Les effets du bruxisme génèrent à la fois un frottement des mâchoires et de sérieuses usures des dents et de nombreux réveils. De plus, l'intensité des forces de bruxisme peut provoquer des maux de tête, des douleurs de la mâchoire, des douleurs de dents, des maux d'oreille sans que les personnes n'en connaissent la cause et cherchent alors l'aide de professionnels de santé.

Il est connu d'utiliser des orthèses buccales dentaires désignées également par gouttières occlusales pour corriger des défauts dentaires ou de posture. En particulier, une gouttière occlusale est utilisée pour diminuer le phénomène consistant à serrer et / ou à grincer les dents pendant le sommeil et cela de façon inconsciente. La gouttière occlusale se place chaque soir sur la denture inférieure. C'est en effet, la mâchoire inférieure qui est mobile et qui a pour rôle d'écraser les aliments contre la mâchoire supérieure fixe, qui sert de billot.

### [Problème technique.]

Cependant, une telle gouttière empêche l'usure des dents, mais n'entrave pas les mouvements inconscients de mastication injustifiée en dehors bien entendu des repas. Or, ces mouvements de la mâchoire perturbent le sommeil.

On pourra se pourra se reporter également à l'état de la technique suivant :

Le document DE 10214800 (D1) décrit un dispositif d'occlusion dentaire. Ce dispositif est maintenu dans la bouche des personnes au moyen d'un autocollant. L'utilisateur fixe le dispositif dans sa bouche par un autocollant à chaque mise en place.

Le document EP0818213 (D2) décrit un dispositif d'occlusion dentaire. Ce dispositif est dans un matériau dur. Le dispositif a une surface supérieure qui épouse la forme du palais de la personne et comporte une bordure périphérique qui se positionne entre les dents du dessus et les dents du dessous. Le maintien dans la bouche est obtenu par cette bordure périphérique qui est munie d'orifices permettant aux dents de se caler.

Le document US2012/0031411 (D3) décrit un dispositif d'occlusion dentaire. Ce dispositif se présente sous forme d'une pince. Le dispositif n'a pas une forme adaptée pour occuper un espace entre la denture supérieure et la denture inférieure et rendre impossible tout contact direct entre les deux dentures.

Le document WO2010/035303 (D4) décrit un dispositif d'occlusion dentaire et se présente sous forme d'une gouttière. Le dispositif n'a pas une forme adaptée pour occuper un espace entre la denture supérieure et la denture inférieure afin de rendre impossible tout contact direct entre les deux dentures.

Le document US 5046512 (D5) décrit un dispositif d'occlusion dentaire. Le dispositif comporte une pièce cylindrique munie d'une rainure pour loger l'extrémité de la langue. Le dispositif comporte des encoches pour loger les dents de devant et être maintenu à l'avant de la bouche entre les dents de devant. Le dispositif n'a pas une forme adaptée pour occuper un espace entre la denture supérieure et la denture inférieure et rendre impossible tout contact direct entre les deux dentures.

La présente invention a pour objet de résoudre ce problème en proposant un dispositif d'occlusion dentaire comportant un corps se présentant sous la forme d'un pavé en matériau souple et ayant une forme qui permet de rendre impossible tout contact direct entre les deux dentures.

### [Brève description de l'invention.]

La présente invention a plus particulièrement pour objet, un dispositif d'occlusion dentaire pour la suppression de l'intensité des forces de bruxisme, principalement caractérisé en ce qu'il comprend un corps comportant un matériau souple, ledit corps se présentant sous la forme d'un pavé de forme adaptée pour occuper un espace entre la denture supérieure et la denture inférieure et rendre impossible tout contact direct entre les deux dentures, le dit pavé est sensiblement parallélépipédique et comprend sur chaque face une proéminence.

Avantageusement, le corps en forme de pavé a une largeur supérieure ou égale à la distance allant d'un bord extérieur des dents situées d'un coté de la denture inférieure /supérieure, à un bord extérieur des dents situées de l'autre coté de la denture inférieure/supérieure et une hauteur supérieure à la hauteur moyenne de la couronne des dents.

Selon d'autres caractéristiques avantageuses du dispositif :
- le corps en forme de pavé est sensiblement parallélépipédique avec sur chaque face une proéminence.
- la proéminence a la forme d'une calotte sphérique ou ellipsoïdale, présente sur chaque face du corps.
- le corps forme un ensemble compact en matériau souple.
- le corps comporte une partie parallélépipédique en matériau dur se prolongeant par une partie sphérique en matériau dur formant une boule et une enveloppe en matériau souple.
- l'enveloppe recouvre la partie avant du corps.
- le corps en forme de pavé est cylindrique et comporte au moins une bordure périphérique supérieure et/ou inférieure comprenant des entailles et/ou empreintes destinées à être en correspondance avec au moins les premières prémolaires.
- le corps cylindrique comporte une face supérieure et une face inférieure incurvées.
- le pavé est en un seul bloc ou formé de plaques superposées et collées de forme adaptée à la forme finale.
- le matériau souple est un matériau caoutchouteux comme du caoutchouc naturel ou synthétique ou du silicone.
- la largeur (L) ou diamètre (d) du corps est de 4cm à 7 cm et de préférence de 5, 5 cm à 6, 5 et encore préféré de 6 cm et la hauteur (H) du corps est de 1 cm à 3 cm, et de préférence de 1, 2cm à 2, 5 cm et encore préféré de 2cm.

### [Description des dessins]

D'autres particularités et avantages de l'invention apparaitront à la lecture de la description suivante faite à titre d'exemple illustratif et non limitatif, en référence aux Figures annexées qui représentent :
- La Figure 1, un schéma d'une vue en perspective d'un premier exemple de réalisation du dispositif selon l'invention,
- La figure 2, un schéma une vue de dessus du dispositif de la figure 1,
- La figure 3, une vue arrière (coté inséré dans la bouche) du dispositif représenté sur les figures 1 et 3,
- La figure 4, un schéma d'une vue en perspective d'un deuxième exemple de réalisation du dispositif selon l'invention,
- La figure 5, un schéma d'une vue suivant une coupe longitudinale AA selon la figure 4,
- La figure 6, un schéma d'une vue en coupe longitudinale suivant une variante d'exécution,

### [Description Détaillée]

On entend par forme adaptée pour occuper l'espace entre la denture supérieure et la denture inférieure, toute forme géométrique de préférence à bords arrondis s'étendant de la denture droite à la denture gauche et couvrant ladite denture au moins à partir des prémolaires jusqu'au incisives, de manière à rendre impossible tout contact direct entre les deux dentures.

On entend par matériau souple, un matériau caoutchouteux. Le matériau caoutchouteux est par exemple un caoutchouc naturel ou de synthèse ou du silicone. Le matériau souple se déforme sous la pression des dents et absorbe les forces exercées par les dents.

On entend par matériau dur un matériau qui ne se déforme pas sous la pression. Il s'agit par exemple d'un plastique dur.

Le dispositif se présente sous une forme compacte comme un pavé. Ce pavé peut être en un seul bloc ou formé de superposition de plaques collées de forme adaptée à la forme finale.

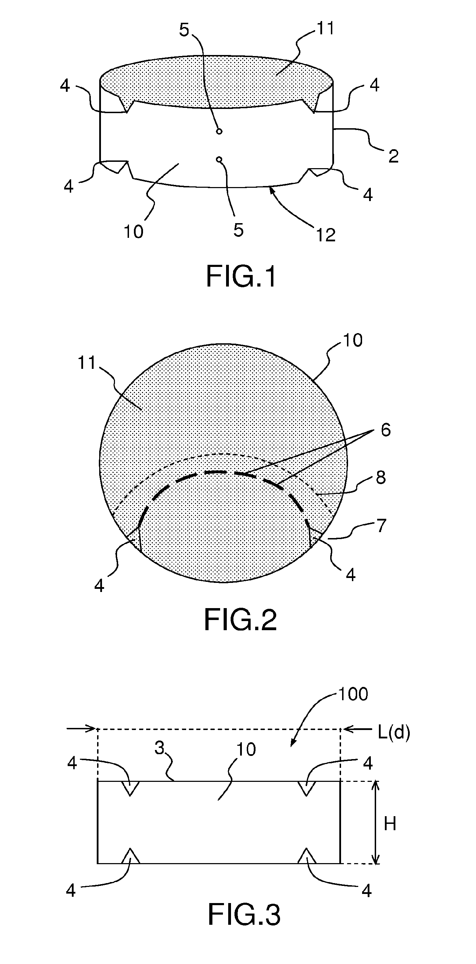

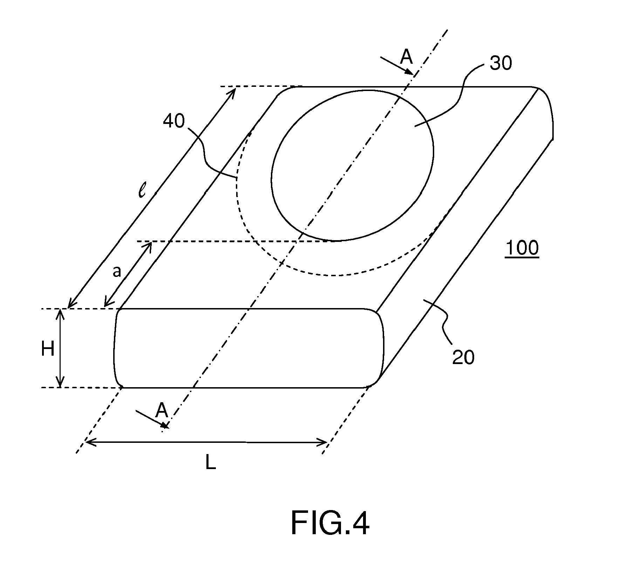

Dans la description qui va suivre on se reportera aux figures décrites ci-dessus qui illustrent deux exemples de réalisation du dispositif selon l'invention. Le dispositif 100 comporte un corps référencé 10 sur la figure 1 et 20 sur la figure 4. Ce corps est en matériau souple et peut être creux ou compact. De préférence le corps 10 ou 20 est dans un matériau compact et forme un pavé.

Le matériau (ou les matériaux) choisi est un matériau inerte chimiquement respectant la réglementation relative aux contacts alimentaires. Ce matériau est choisi avantageusement parmi les organopolysiloxanes. Il peut comporter éventuellement des charges, colorants figurant dans une liste de produits autorisés par la réglementation sur les produits à contact alimentaires. Avantageusement le matériau souple est de préférence en caoutchouc ou en silicone.

Avantageusement, selon un premier mode de réalisation, le corps 10 a une forme cylindrique comme représenté par les figures 1 à 3. Selon un deuxième mode de réalisation, le corps 20 et a une forme allongée sensiblement parallélépipédique comme représenté par les figures 4 à 6.

Sur les figures 1, 2 et 3, le dispositif 100 comporte un corps 10 en matériau souple de forme cylindrique. Par convention, on désigne face supérieure 11, la base du cylindre destinée à être en regard avec le palais et la denture supérieure d'une personne qui met en place le dispositif dans sa bouche et par face inférieure 12, la base du cylindre destinée à être en contact avec la langue de la dite personne et la denture inférieure. Les bordures des faces supérieure 11 et inférieure 12 comportent des entailles 4 disposées à l'emplacement prévu pour les première prémolaires.

Le corps cylindrique 10 est introduit dans la bouche jusqu'à ce que les premières prémolaires rencontrent les entailles 4 comme illustré par la figure 2. Ces entailles permettent de caler /fixer le corps 10 dans la bouche après un léger serrage des mâchoires. Le pointillé 8 illustre le contact des lèvres sur le corps 10.

Avantageusement, les deux bases 11, 12 du corps 10 ne sont pas plates, mais incurvées vers l'intérieur, le niveau le plus bas porte la référence 5 sur la figure 1. Ainsi, les quatre incisives, très sensibles, ne sont qu'en léger contact avec le corps souple 10 à l'emplacement symbolisé par la ligne en pointillés 6 sur cette figure 1, dessus et dessous, et la langue trouve sa place dessous. Les premières prémolaires symbolisées par la référence 7 sur la figure 2, prennent leur place dans les entailles 4. Les lèvres (non représentées) sont en contact avec le caoutchouc 8, dessus et dessous.

Le diamètre du corps 10 est de préférence de 4 cm à 7 cm et de préférence de 5, 5 cm à 6, 5 et encore préféré de 6 cm.

La hauteur du corps 10 est de 1 cm à 3 cm, et de préférence de 1, 2 cm à 2, 5 cm et encore préféré de 2 cm.

Lorsque le dispositif est en place dans la bouche, les mouvements latéraux de la mastication inconsciente qui déplacent la mâchoire inférieure à gauche ou à droite dans le phénomène du bruxisme sont alors ainsi éliminés.

Dans le mode de réalisation du dispositif représenté sur les figures 4, 5 et 6, le dispositif a une forme différente. Cette forme permet de maintenir ledit dispositif 100 en place dans la bouche. Le dispositif se présente sous forme d'un pavé sensiblement parallélépipédique comportant une proéminence 30. La présence d'une proéminence 30 comme illustré sur les figures permet de caler le dispositif I00 dans la bouche et ainsi de le maintenir malgré le fait que la bouche soit à ce stade presqu'à moitié ouverte. Même si la bouche s'ouvre inconsciemment davantage pendant le sommeil, le maintien du dispositif 100 entre les deux mâchoires est toujours assuré du fait de la présence de la proéminence 30.

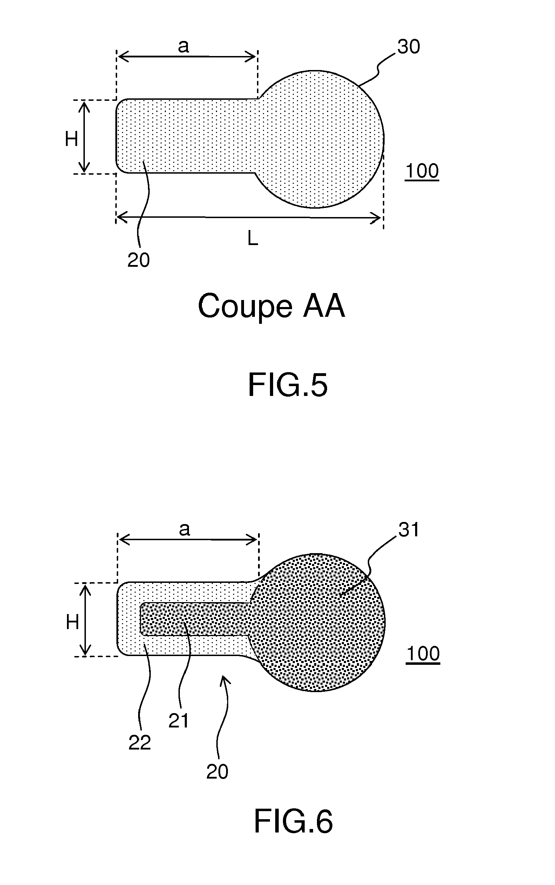

Ainsi, selon ce deuxième mode de réalisation, le dispositif 100 comporte un corps 20 de forme allongée sensiblement parallélépipédique muni d'une proéminence 30 sur chaque face. La proéminence 30 se présente sous forme d'une calotte sphérique présente sur les faces supérieure et inférieure du corps 100. Le diamètre des calottes sphériques 32 est de 40 à 45 mm. L'emplacement de la denture est illustré par le pointillé 40 sur les faces supérieure et inférieure du corps 20. La longueur a de la partie avant du corps au delà de la calotte, est de 25 à 35 mm de préférence 30mm. Comme dans l'exemple décrit précédemment, le corps 20 est en matériau souple, avantageusement en caoutchouc ou silicone. La figure 5 illustre une coupe AA selon la figure 4. Les faces supérieure et inférieure comportent une proéminence sous forme d'une calotte.

La figure 6 illustre une vue en coupe AA selon une variante d'exécution. Dans cette variante le dispositif 100 comporte un corps 20 sensiblement parallélépipédique comportant une partie parallélépipédique 21 en matériau dur se prolongeant par une partie sphérique 31 en matériau dur formant une boule 31 et une enveloppe en matériau souple 22. La boule 31 est de préférence en matériau plastique dur. L'enveloppe 22 est avantageusement en caoutchouc ou en silicone. Dans cet exemple de réalisation, l'enveloppe 22 recouvre la partie avant 21 du corps 20. Il peut être envisagé selon un autre exemple non représenté, que l'enveloppe 22 recouvre la totalité du corps 20, venant ainsi recouvrir également la boule 31. Le diamètre de la boule sphérique 32 est de 40 à 45 mm.

Le corps a une longueur l, une largeur L et une hauteur H.

La largeur L du corps 20 est de préférence de 4cm à 7cm et de préférence de 5,5 cm à 6,5 cm et encore préféré de 6 cm.

La hauteur H du corps est de préférence de 1 cm à 3 cm, et de préférence de 1,2 cm à 2, 5 cm et encore préféré de 2cm.

Dans les deux exemples de réalisation relatifs aux figures 1 à 5, le pavé peut être réalisé en un seul bloc caoutchouteux ou par superposition de plaques caoutchouteuses collées entre elles. Les plaques ont une épaisseur de quelques millimètres de 1 mm à 5mm.

Pour l'exemple illustré sur la figure 6, l'enveloppe 22 est collée sur la partie avant 21 et en partie sur la boule 31.

Avec le dispositif décrit dans les différents modes de réalisation et variantes d'exécution, les mouvements masticatoires sont supprimés, ainsi que les frottements des dentures et les réveils en sursaut quand la mâchoire se met à trembler fortement. Ainsi, l'appui des lèvres sur le caoutchouc supprime totalement ou presque l'entrée d'air par la bouche. Ce qui est conforme à ce qu'a prévu la nature, laquelle ne déclenche la respiration buccale qu'au cours d'efforts musculaires intenses et soutenus, afin de porter secours aux muscles thoraciques qui doivent augmenter le débit d'oxygène dans les poumons. Il est très facile, en cas de besoin, d'éjecter le dispositif 100, en ouvrant totalement la mâchoire inférieure et en poussant le corps 10, 20 avec la langue, comme le fait inconsciemment un bébé pour éjecter la tétine retenue dans sa bouche, si sa respiration nasale devient insuffisante.

Ce dispositif anti-bruxisme ne verrouille donc pas la respiration buccale si besoin est. En revanche, il supprime totalement ou presque le ronflement buccal, très sonore.

Des essais ont été faits en introduisant le dispositif dans un sac de matière plastique destiné à congeler les aliments, afin de ne pas mettre les muqueuses de la bouche en contact direct avec le matériau souple du corps 10, 20. Ces sacs n'ont pas été percés, même au bout d'une quinzaine de nuits. Ainsi, on a pu constater que les frottements entre mâchoires sont supprimés.

En outre, la suppression de la respiration buccale du fait de la présence du dispositif dans la bouche, diminue fortement le débit d'air dans les poumons ce qui diminue l'inhalation de pollen et de particules nocives présentes dans la poussière des pièces.

Le dispositif permet en outre de diminuer les risques liés à l'apnée du sommeil.

L'utilisation du dispositif selon l'invention est très facilement supporté sans contrainte par les utilisateurs quand ils utilisent déjà un dispositif destiné à traiter les apnées du sommeil. Le bruxisme et l'apnée du sommeil sont liés. En effet, éliminer la respiration buccale nécessite un supplément d'énergie pour gonfler le thorax et les poumons afin d'imposer une nouvelle distribution de l'air inspiré. Or un petit appareil générant de l'air soufflé génère déjà la légère surpression qui gonfle les poumons.

## Revendications

1. Dispositif d'occlusion dentaire pour la suppression de l'intensité des forces de bruxisme, **caractérisé en ce qu'**il comprend un corps (10, 20) comportant un matériau souple, ledit corps se présentant sous la forme d'un pavé de forme adaptée pour occuper un espace entre la denture supérieure et la denture inférieure et rendre impossible tout contact direct entre les deux dentures ; le dit pavé est sensiblement parallélépipédique et comprend sur chaque face une proéminence (30).

2. Dispositif d'occlusion selon la revendication 1, **caractérisé en ce que** le corps en forme de pavé a une largeur supérieure ou égale à la distance allant d'un bord extérieur des dents situées d'un côté de la denture inférieure ou supérieure, à un bord extérieur des dents situées de l'autre côté de la denture inférieure ou supérieure et une hauteur supérieure à la hauteur moyenne de la couronne des dents.

3. Dispositif d'occlusion selon la revendication 1, **caractérisé en ce que** la proéminence (30) a la forme d'une calotte sphérique ou ellipsoïdale, présente sur chaque face du corps (20).

4. Dispositif d'occlusion selon l'une quelconque des revendications précédentes, **caractérisé en ce que** le corps (20) forme un ensemble compact en matériau souple.

5. Dispositif d'occlusion selon les revendications 1ou 3, **caractérisé en ce que** le corps (20) comporte une partie parallélépipédique (21) en matériau dur se prolongeant par une partie sphérique (31) en matériau dur formant une boule et une enveloppe en matériau souple (22).

6. Dispositif d'occlusion selon la revendication 5, caractérisé l'enveloppe (22) recouvre la partie avant (21) du corps (20).

7. Dispositif d'occlusion selon la revendication 1, **caractérisé en ce que** le matériau souple est un matériau caoutchouteux comme du caoutchouc naturel ou synthétique ou du silicone.

8. Dispositif d'occlusion selon l'une quelconque des revendications précédentes, **caractérisé en ce que** la largeur (L) ou diamètre (d) du corps est de 4cm à 7 cm et de préférence de 5, 5 cm à 6, 5 et encore préféré de 6 cm et **en ce que**, la hauteur (H) du corps est de 1 cm à 3 cm, et de préférence de 1, 2cm à 2, 5 cm et encore préféré de 2cm.
